# EUROPEAN PATENT APPLICATION

(11) **EP 2 174 656 A1**
(43) Date of publication of application: **14.04.2010**
(21) Application number: 08787632.2
(22) Date of filing: 25.06.2008
(51) Int. Cl.: A61K 9/50, C08B 37/04, A61P 7/06

(54) **MICROPARTICLES OF ALGINATE MODIFIED WITH RGD AS A RELEASE SYSTEM FOR MEDICINES**

(30) Priority: 26.06.2007 ES 200701790
(71) Applicant: Universidad del Pais Vasco, 48940 Leioa Vizcaya (ES)
(72) Inventor: ORIVE ARROYO, Gorka, E-48940 Leioa (ES); PEDRAZ MUÑOZ, José Luis, E-48940 Leioa (ES); HERNÁNDEZ MARTÍN, Rosa María, E-48940 Leioa (ES)
(74) Representative: ABG Patentes, S.L.
(86) International application number: PCT/ES2008/000451
(87) International publication number: WO 2009/000955

(57) **Abstract**

The object of the present invention is to provide particles of a polymeric material containing cells therein, wherein said particles have a strength that is substantially greater than the microcapsules known in the state of the art. Said strength is achieved by means of functionalizing the polymeric material forming the microcapsule with a peptide which can bind to the adhesion proteins of the cell membrane, such that the cells act as cross-linking agents of the matrix resulting in an improvement of the mechanical properties of the matrixes

## Description

### Technical Field of the Invention

The present invention relates to encapsulated cell systems and, more specifically, to microcapsules formed by a polymeric material which has been modified by a peptide which is capable of binding to the adhesion proteins found in the cell membrane such that the cells that are inside the microcapsule interact with said peptide. The invention also contemplates microcapsules in which the cells have been genetically modified to express a protein of therapeutic interest as well as the therapeutic applications of said microcapsules.

### Background of the Invention

Cell microencapsulation technology is based on the immobilization of cells within a polymeric matrix surrounded by a semi-permeable membrane. Said encapsulated cells are protected against the rejection of the cell and antibody-dependent immune system and have the potential of producing a wide range of therapeutically active substances (Orive, G, et al. 2003, Nat. Med. 9:104-107, Orive, G. Trends Biotechnol. 2004, 22:87-92).

These "live" drug release systems are especially interesting for the controlled and continued expression of hormones and growth factors, for the local and targeted release of drugs and for the improvement of the pharmacokinetics of easily degradable peptides and proteins, which frequently have a short *in vivo* half-life.

Due to its characteristics, alginate has frequently been used as a microencapsulation material. Thus, Orive, G. et al. (Mol. Therapy, 2005, 12:283-289) describe alginate microcapsules coated with a semi-permeable poly-L-lysine membrane containing genetically modified myoblasts and expressing erythropoietin (EPO). The alginate microcapsules are stabilized by means of divalent cations which, after their implantation in the organism, tend to spread, resulting in a progressive weakening of the structure of the microcapsule. Until now, the attempts to improve the long-term mechanical strength of these systems have been based on reinforcing the microcapsules with a semi-permeable membrane (De Castro, M. et al. 2005, J Microencapsul. 22(3):303-15), by means of the use of variants of alginate modified by means of polyelectrolyte condensation with another polymer, such as poly(methylene-co-guanidine) (Orive, G. et al. 2003, Int.J.Pharm. 18:57-68) or by means of the cross-linking of alginate molecules with an ethylenic monomer (WO03/094898). However, this type of modified polymer does not provide a suitable microenvironment for cell viability. US patent application US2006/0251630 describes agarose microcapsules containing encapsulated cells in which the microcapsules have been prepared in the presence of fibrinogen. However, the microcapsules are intended for the quick release of the cells, they are therefore capsules lacking high stability. Markusen, J.F. et al. (Tissue Engineering, 2006, 12:821-830) describe alginate capsules modified with a GRGDY peptide and containing adult mesenchymal cells, as well as their use as tissue replacements. However, this study does not demonstrate that the encapsulated cells survive for more than 15 days from the moment of implantation.

Therefore there is a need in the art for alginate microcapsules having greater stability and in turn assuring long-term viability of the cells contained therein.

### Summary of the Invention

In a first aspect, the invention relates to a particle with a maximum diameter of 1 mm comprising:
a) a polymer X functionalized with at least one peptide Y;
b) at least one cell having on its surface specific binding sites for said peptide Y, wherein the cell is bound to polymer X by means of the interaction between peptide Y and the specific binding sites for said peptide Y on the surface of the cell.

In a second aspect, the invention relates to a microcapsule comprising a particle of the invention surrounded by a semi-permeable membrane.

In another aspect, the invention relates to a particle or microcapsule of the invention for the use thereof in medicine.

In another aspect, the invention relates to the use of the particle or microcapsule of the invention for preparing a medicinal product for the treatment and prevention of diseases which require a supply of the peptide compound with biological activity which is expressed by the cells forming part of the microcapsules.

In another aspect, the invention relates to the use of the particle or microcapsule of the invention as a device for the release of a peptide compound with biological activity.

In another aspect, the invention relates to a method for producing a particle of the invention comprising the steps of
a) Contacting a polymeric substance which is modified with a peptide Y with at least one cell having on its surface specific binding sites for said peptide Y and
b) applying conditions allowing the formation of particles of polymeric material having less than 1 mm in diameter.

In another aspect, the invention relates to a method for preparing a microcapsule of the invention comprising the steps of
a) Contacting a polymeric substance which is modified with a peptide Y with cells having the capacity to bind to said peptide Y
b) applying conditions allowing the formation of particles of polymeric material having less than 1 mm in diameter
c) coating the microparticle formed in step (b) with a membrane made of a material different from the polymer used in step (a).

### Brief Description of the Drawings

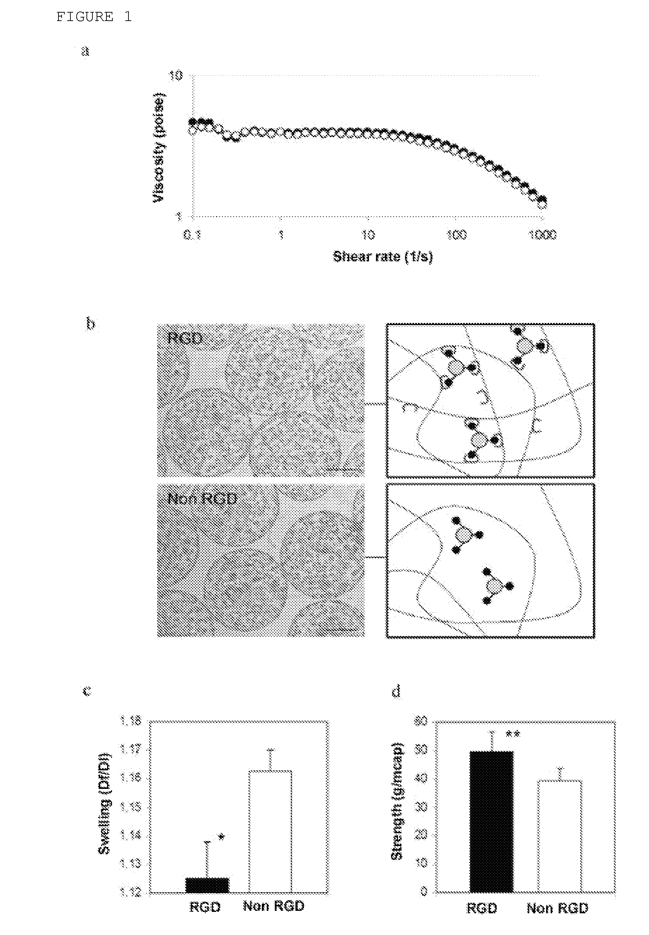

Figure 1. a) viscosity of the alginates-RGD mixture and the LVG alginate solution. b) Illustration describing the effect of the bioactivation of the alginate matrixes with RGD, which facilitates the adhesion of the immobilized cells to the matrix; c) Study of the osmotic stability of the LVG capsules compared to the capsules bioactivated with RGD; d) Mechanical strength by compression of both particles by means of texture profile analysis.

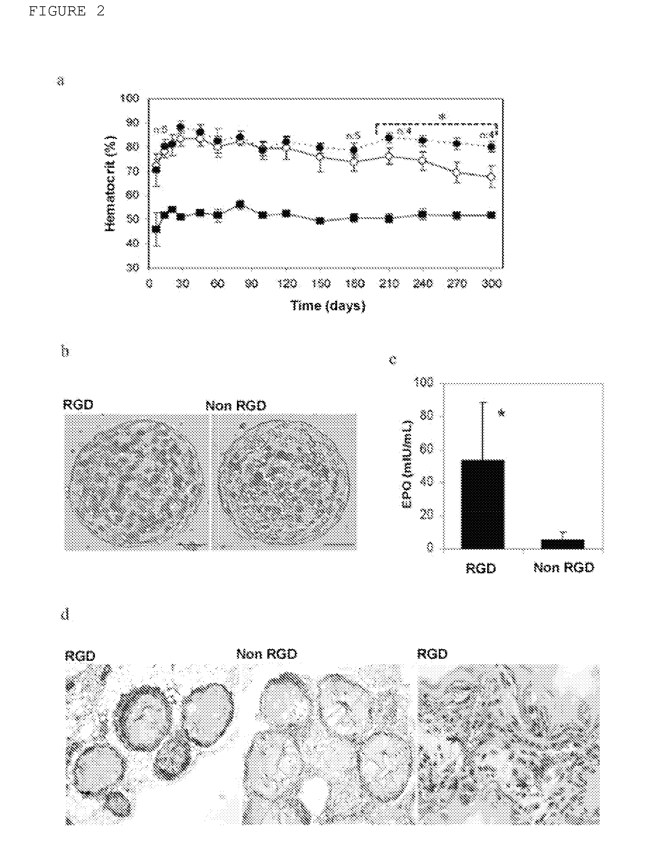

Figure 2. a) Follow-up for 300 days of the hematocrit of the animals implanted with LVG microcapsules and microcapsules bioactivated with RGD compared to control animals; b) Microscopic image of capsules extracted on day 300 after starting treatment; c) Production of EPO of the capsules explanted on day 300 from the animals; d) Histology of the microcapsules on day 300.

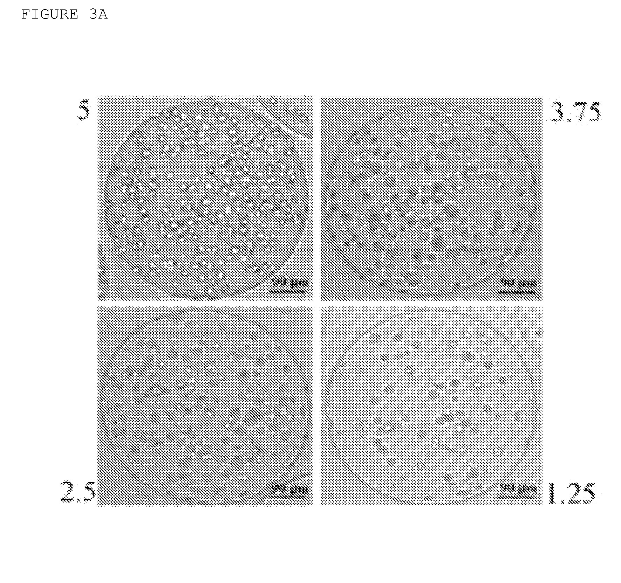

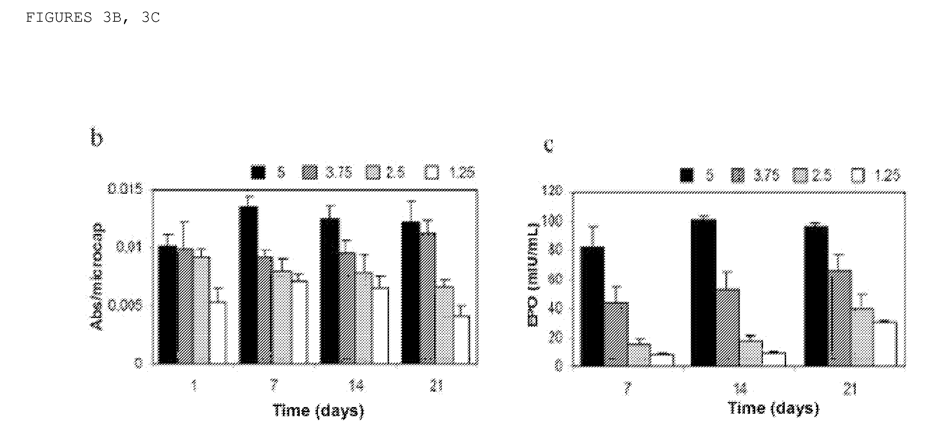

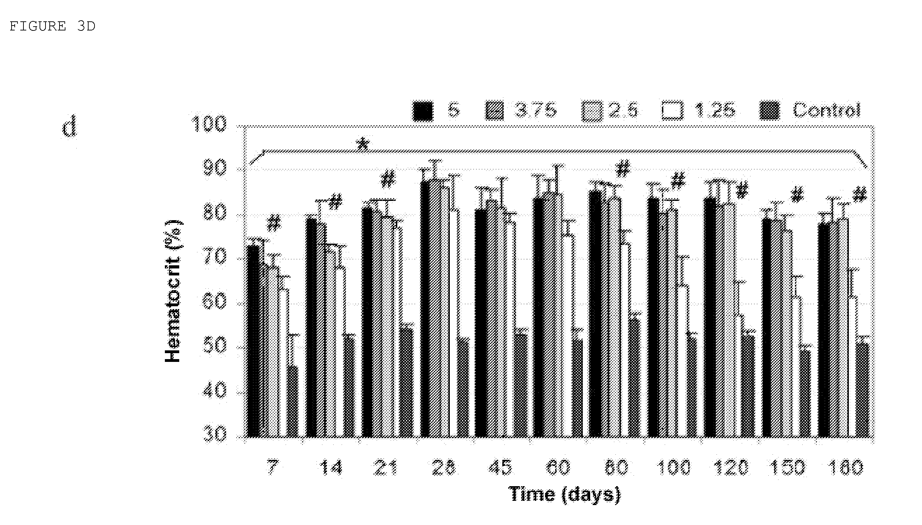

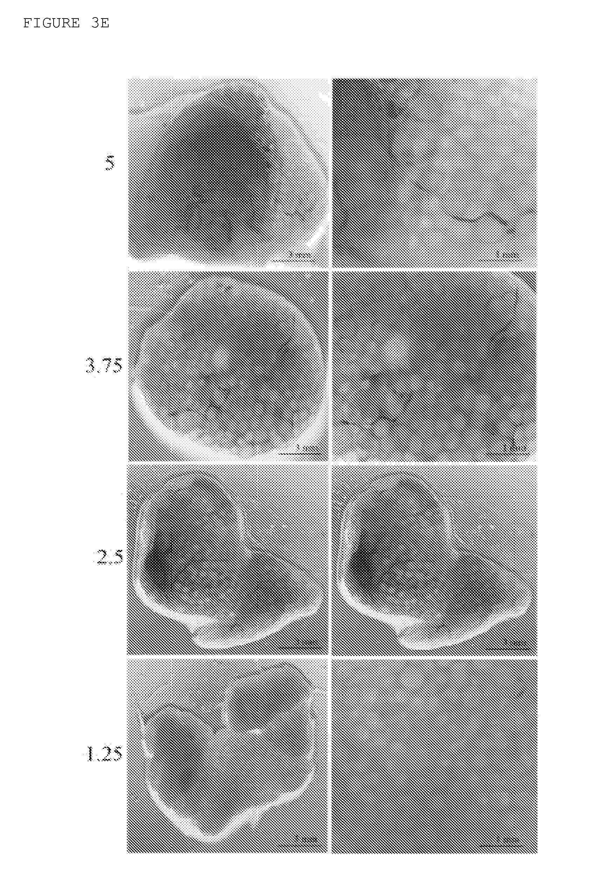

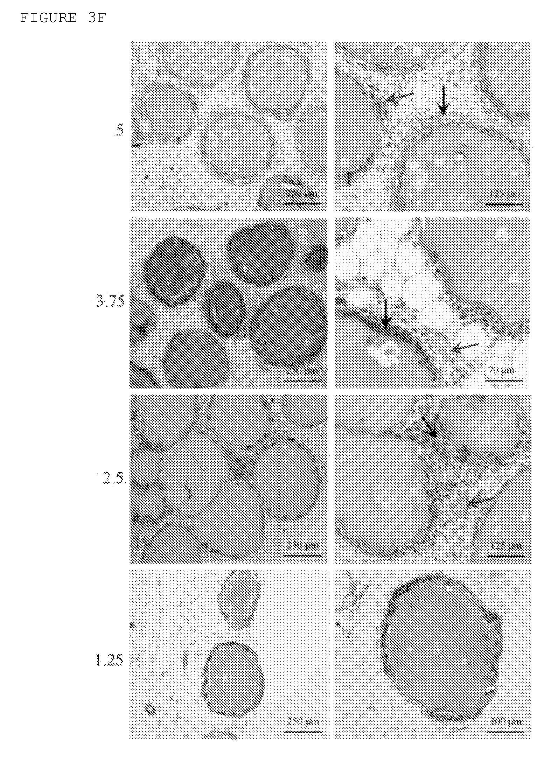

Figure 3: Dose-response study: a) Initial images of the RGD microcapsules with different cell densities; b) and c) viability and production of EPO of the encapsulated cells, respectively; d) viability of the doses of capsules at different cell densities for 6 months; e) illustrations corresponding to the microcapsule aggregates and the induced vascularization at 6 months; f) Histology of the different microcapsules

### Detailed Description of the Invention

The authors of the present invention have surprisingly observed that when alginate microparticles containing cells are prepared with an alginate which has been modified by means of conjugation with a peptide with cell bioadhesion properties, the resulting microparticles have a physical strength that is much greater than microcapsules containing alginate which is not modified. Without intending to be bound by any theory, it is believed that this effect is due to the occurrence of interactions between the adhesion molecules which are on the surface of the microencapsulated cells and the adhesion peptides coupled to the alginate, which results in the cells being incorporated into the matrix as cross-linking agents, resulting in improved mechanical properties of the matrixes. The inventors have also observed that the viability of the cells in the particles can be compromised if the particles are larger than 1 mm in diameter. Without wishing to be bound by any theory, it is believed that the small diameter of the particles allows the cells to be located at a maximum distance of 100 to 200 µm with respect to the source of nutrients (usually a capillary), which assures an optimal supply of nutrients.

To that end, in a first aspect the invention relates to a particle with a maximum diameter of 1 mm comprising:
a) a polymer X functionalized with at least one peptide Y and
b) at least one cell having on its surface specific binding sites for said peptide Y, wherein the cell is bound to polymer X by means of the interaction between peptide Y and the specific binding sites for said peptide Y on the surface of the cell.

The exact diameter of the particles is not limiting provided that it is less than 1 mm. Therefore in preferred embodiments, the particles have a maximum diameter of between 1 and 0.9, between 0.9 and 0.8, between 0.8 and 0.7, between 0.7 and 0.6, between 0.6 and 0.5, between 0.5 and 0.4, between 0.4 and 0.3, between 0.3 and 0.2, between 0.2 and 0.1 or less than 0.1 mm.

Filler materials suitable to act as a support of the cells can be any type of polymeric material, particularly thermoplastic polymers or hydrogel polymers.

Included among hydrogel type polymers are natural materials such as alginate, agarose, collagen, starch, hyaluronic acid, bovine serum albumin, cellulose and derivatives thereof, pectin, chondroitin sulfate, fibrin and fibroin, as well as synthetic hydrogels such as sepharose and sephadex.

Included among thermoplastic polymers are acrylic acid, acrylamide, 2-aminoethyl methacrylate, poly(tetrafluoroethylene-co-hexafluoropropylene), methacrylic-acid-(7-coumaroxy) ethyl ester, N-isopropyl acrylamide, polyacrylic acid, polyacrylamide, polyamidoamine, poly(amino)-p-xylylene, poly(chloroethylvinylether), polycaprolactone, poly(caprolactone-co-trimethylene carbonate), poly(carbonate urea) urethane, poly(carbonate) urethane, polyethylene, polyethylene and acrylamide copolymer, polyethyleneglycol, polyethyleneglycol methacrylate, poly(ethylene terephthalate), poly (4-hydroxybutyl acrylate), poly (hydroxyethyl methacrylate), poly(N-2-hydroxypropyl methacrylate), poly(lactic acid-glycolic acid), poly(L-lactic acid), poly(gamma-methyl, L-glutamate), poly(methyl methacrylate), poly(propylene fumarate), poly(propylene oxide), polypyrrole, polystyrene, poly(tetrafluoroethylene), polyurethane, polyvinyl alcohol, ultra-high molecular weight polyethylene, 6-(p-vinylbenzamido)-hexanoic acid and N-p-vinylbenzyl-D-maltonamide and copolymers containing more than one of said polymers.

In a preferred embodiment, the polymer used as a support in the microcapsules of the invention is alginate. In theory, any type of alginate capable of forming a hydrogel is suitable for being used in the microcapsules of the invention. Therefore, the microcapsules of the invention can contain alginate mostly formed by mannuronic acid regions (MM blocks), by guluronic acid regions (GG blocks) and by mixed sequence regions (MG blocks). The percentage and distribution of the uronic acids differ according to the origin of the alginate and contribute to the properties of the alginate. The person skilled in the art knows the percentages of each of the different blocks appearing in the different biological sources of the alginates. Thus, the invention contemplates the use of alginates from *Laminaria hyperborea, Lessonia nigrescens, Lessonia trabeculata, Durvillaea antarctica, Laminaria digitata, Ecklonia maxima, Macrocystis pyrifera, Ascophyllum nodosum* and/or *Laminaria japonica* as well as mixtures of alginates of different species until achieving the desired content of GG, MM or GM blocks. The GG blocks contribute to the rigidity of the hydrogel, whereas the MM monomers maintain a high tensile strength, such that by means of the use of a suitable combination of alginate polymers, a mixture can be obtained with an elastic modulus of a suitable value whereas the viscosity of the pre-gel solution is kept at low enough levels to allow suitable handling and cell immobilization. Thus, the alginates which can be used in the present invention include GG alginates, MM alginates or combinations of both at a ratio of 90:10, 80:20, 70:30, 60:40, 50:50, 40:60, 30:70, 20:80 or 10:90. In a preferred embodiment, a mixture of both types of polymers has been used to form binary hydrogels containing 50% irradiated MVG polymers and 50% MVG-type polymers substituted with RGD sequences.

Additionally, the invention also contemplates the use of alginates derived from the treatment of natural alginates with enzymes which are capable of modifying the integrating blocks to give rise to alginates with improved properties. Thus, alginates resulting from treating alginates with C5-epimerases, converting M blocks into G blocks, as well as with the enzyme AlgE4 of the bacteria *Azotobacter vinelandii*, which is capable of converting relatively rigid M blocks into MG blocks. The invention alternatively contemplates the use of alginates which have been modified by different physical treatments, particularly gamma rays, irradiation with ultrasound or with ultraviolet light as described by Wasikiewicz, J.M. et al. (Radiation Physics and Chemistry, 2005, 73:287-295). Thus, in a preferred embodiment the alginates have been treated with a source of gamma rays, preferably Co⁶⁰, at a dose of 10-500 kGy. The treatment with gamma rays results in the degradation of the alginate, whereby the GG, MM or GM alginate blocks which are incorporated in the particle have a lower molecular weight.

The polymer forming part of the microcapsules is functionalized with a peptide Y, which allows the binding of the cells which are in the microcapsules with the polymer through the specific interaction between components of the cell membrane and peptide Y. In principle, any peptide having specific binding sites in the outside of the cell membrane can be used in the present invention. Thus, peptide Y can come from cell adhesion molecules interacting with the extracellular matrix such as fibronectin, the different members of the families of the selectins, cadherins, lectins, integrins, immunoglobulins, collectins and galectins.

In a preferred embodiment, peptide Y is a peptide derivative of a region selected from the regions of the fibronectin involved in the binding with the integrins located in the cell membrane. Said peptides preferably derive from the region of the tenth type III repeat of fibronectin containing the RGD peptide, from the region of the fourteenth type III repeat of the fibronectin containing the IDAPS peptide, from the CSI region of fibronectin containing the LDV peptide and the CS5 region of the fibronectin containing the REDV peptide. These peptides can consist of fragments of the corresponding regions which conserve their adhesive capacity, such as for example, the QAGDV peptide of fibrinogen, the LDV peptide of fibronectin and the IDSP peptide of VCAM-I. In a particular embodiment, said integrin-binding peptide is a peptide derivative of the tenth type III repeat of fibronectin comprising the RGD sequence, such as, for example, a peptide selected from the group of RGD, RGDS, GRGD, RGDV, RGDT, GRGDG, GRGDS, GRGDY, GRGDF, YRGDS, YRGDDG, GRGDSP, GRGDSG, GRGDSY, GRGDVY, GRGDSPK, CGRGDSPK, CGRGDSPK, CGRGDSY, cyclo(RGDfK), YAVTGRGD, AcCGGNGEPRGDYRAY-NH2, AcGCGYGRGDSPG and RGDPASSKP, cyclic variants of said peptides, both linear and branched multivalent variants (see for example Dettin, M. et al. 2002, J. Biomed. Mater. res. 60:466-471, Monaghan, S. et al. 2001, Arkivoc, 2:U42-49, Thumshirn, G. et al. 2003, Chemistry 9:2717-25, Scott, E.S. et al, 2001, J.Gene Med. 3:125-134)) as well as combinations of two or more of said peptides.

The cell adhesion peptide can be bound to the polymer acting as a support through the N-terminal end or the C-terminal end and, independently of the anchor point, it can be bound directly to the polymer acting as a support or, alternatively, it can be bound through a spacing element. Virtually any peptide with structural flexibility can be used. By way of illustration, said flexible peptide can contain repeats of amino acid residues, such as (Gly)₄, Gly-Gly-Gly-Ser, (Gly)₁₃ (Beer, J.H. et al., 1992, Blood, 79, 117-128), SPASSKGGGGSrL6-NH2 (Craig, W.S. et al. 1995, Biopolymers, 37:157-175) or any other suitable repeat of amino acid residues, or the hinge region of an antibody.

The invention likewise contemplates different degrees of substitution of the alginate with the bioadhesive peptide, such that during the process for conjugating the polymer with the bioadhesion peptide, the concentrations of both components can vary such that the resulting modified polymer has the desired number of coupled bioadhesive peptides. Thus, the invention contemplates alginates containing between 1 and 100, between 100 and 200, between 200 and 300, between 300 and 400, between 400 and 500, between 500 and 600, between 600 and 700, between 700 and 800, between 800 and 900 and between 900 and 1000 bioadhesive peptide molecules for each polymer molecule.

The particles of the invention can be used as such. However, alginate is a rather unstable polymer which tends to lose calcium and, therefore, to lose its gel nature. Furthermore, the alginate particles are relatively porous, which results in antibodies being able to access their interior and damage the cells. For these reasons, it is appropriate for the microparticles to be surrounded by a semi-permeable membrane conferring stability to the particles and forming an impermeable barrier to antibodies. For this reason, in another aspect the invention relates to microcapsules comprising a particle according to the invention surrounded by a semi-permeable membrane.

Semi-permeable membrane is understood to be a membrane which allows the entrance of all the solutes necessary for cell viability and, in the event that cells producing a therapeutic protein are used, allowing the exit of the therapeutic proteins produced by the cells, but which is substantially impermeable to antibodies, such that the cells are protected from the immune response produced by the organism housing the microcapsules.

Suitable materials for forming the membrane are materials that are insoluble in biological fluids, preferably polyamino acids, such as for example poly-L-lysine, poly-L-ornithine, poly-L-arginine, poly-L-asparagine, poly-L-aspartic, poly benzyl-L-aspartate, poly-S-benzyl-L-cysteine, poly-gamma-benzyl-L-glutamate, poly-S-CBZ-L-cysteine, poly-ε-CBZ-D-lysine, poly-δ-CBZ-DL-ornithine, poly-O-CBZ-L-serine, poly-O-CBZ-D-tyrosine, poly(γ-ethyl-L-glutamate), poly-D-glutamic, polyglycine, poly-γ-N-hexil L-glutamate, poly-L-histidine, poly(α,β-[N-(2-hydroxyethyl)-DL-aspartamide]), poly-L-hydroxyproline, poly(α,β-[N-(3-hydroxypropyl)-DL-aspartamide]), poly-L-isoleucine, poly-L-leucine, poly-D-lysine, poly-L-phenylalanine, poly-L-proline, poly-L-serine, poly-L-threonine, poly-DL-tryptophan, poly-D-tyrosine or a combination thereof, more preferably, polyamino acids with polycationic polyamino acids, even more preferably, the polycationic polyamino acids are poly-L-lysine, poly-L-ornithine and poly-D lysine.

The membrane coating the microcapsule is usually made of a polycationic material, which gives rise to the formation of a polyanion-polycation complex contributing to the stabilization of the alginate and to reducing the porosity of the microcapsule and to forming an immunological barrier impermeable to antibodies. However, the positive charge of said membrane favors cell adhesion to the surface of the microcapsule, resulting in a lower biocompatibility of the microcapsule. Therefore, in a particular embodiment the membrane surrounding the microcapsule is in turn surrounded by a second membrane mostly formed by a material inhibiting cell adhesion. In a preferred embodiment, said material forming the second membrane is an alginate.

Any eukaryotic cell can be used in the present invention provided that it has binding sites for peptide Y on its surface, but mice, rat, primate and human cells are preferred. Thus, cells suitable for carrying out the invention are cardiomyocytes, endothelial cells, epithelial cells, lymphocytes (B and T cells), mastocytes, eosinophils, cells of the vascular intima, primary cultures of cells isolated from different organs, preferably of cells isolated from the islets of Langerhans, hepatocytes, leukocytes, including mononuclear leukocytes, embryonic stem cells, mesenchymal stem cells, umbilical cord stem cells or adult stem cells (from the skin, lung, kidney and liver), osteoclasts, chondrocytes and other cells of the connective tissue. Cells of established lines such as Jurkat T cells, NIH-3T3 cells, CHO cells, Cos cells, VERO cells, BHK cells, HeLa cells, COS cells, MDCK cells, 293 cells, 3T3 cells, C2C12 myoblasts and W138 cells are also suitable cells.

In principle, the number of cells which must form part of the microcapsules is not essential for the invention provided that there is a sufficient number of cells so that they contribute to forming the cross-link and the desired effect of increasing the mechanical strength of the microparticles is obtained. This, in a preferred embodiment the amount of cells for each mL of alginate solution is between 1 and 10 x 10⁶, preferably between 2 and 9 x10⁶, more preferably between 3 and 8 x 10⁶, still more preferably between 4 and 7 x 10⁶ and still more preferably between 5 and 6 x 10⁶. Preferably, the number of cells in the initial mixture is 5; 3.75; 2.5 or 1.25 x 10⁶ for each mL of alginate solution.

In a preferred embodiment, the cells forming part of the microcapsules have been genetically modified such that they produce therapeutic proteins. In this case, the cells must be capable of modulating the expression of the inserted sequences and of modifying and processing the product such that the protein acquires its native conformation. Examples of proteins which can be produced by the cells forming part of the microcapsules object of the invention are erythropoietin (EPO), corticotropin-releasing hormone (CRH), growth hormone-releasing hormone (GHRH), gonadotropin-releasing hormone (GnRH), thyrotropin-releasing hormone (TRH), prolactin-releasing hormone (PRH), melatonin-releasing hormone (MRH), prolactin-inhibiting hormone (PIH), somatostatin, adrenocorticotropic hormone (ACTH), somatotropin or growth hormone (GH), luteinizing hormone (LH), follicle-stimulating hormone (FSH), thyrotropin (TSH or thyroid-stimulating hormone), prolactin, oxytocin, anti-diuretic hormone (ADH or vasopressin), melatonin, Müllerian inhibiting factor, calcitonin, parathyroid hormone, gastrin, cholecystokinin (CCK), secretin, insulin-like growth factor type I (IGF-I), insulin-like growth factor type II (IGF-II), atrial natriuretic peptide (ANP), human chorionic gonadotropin (HCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), tumor necrosis factor-alpha (TNF-α), interleukin 6 (IL-6), interleukin 8 (IL-8 and chemokines), interleukin 12 (IL-12), interleukin 16 (IL-16), alpha, beta, gamma interferons, neuronal growth factor (NGF), platelet-derived growth factor (PDGF), transforming growth factor-beta (TGF-beta), bone morphogenetic proteins (BMPs), fibroblast growth factors (FGF and KGF), epidermal growth factor (EGF and the like), vascular endothelial growth factor (VEGF), granulocyte colony-stimulating factor (G-CSF), glial growth factor, keratinocyte growth factor, endothelial growth factor, alpha 1-antitrypsin, tumor necrosis factor, granulocyte/macrophage colony-stimulating factor (GM-CSF), cyclosporine, fibrinogen, lactoferrin, tissue plasminogen activator (tPA), chymotrypsin, immunoglobulins, hirudine, superoxide dismutase, imiglucerase.

The polypeptides encoding for proteins of therapeutic interest are associated to sequences regulating the expression of said polypeptides. These sequences can be sequences regulating transcription, such as constitutive or inducible promoters, enhancers, transcriptional terminators and sequences regulating translation, such as non-translated localized 5' or 3' sequences with respect to the encoding sequences.

The proteins expressed by the cells forming part of the microcapsules of the invention can be expressed in a transient manner or in a stable manner. In the event that the microcapsules remain in the patient for a prolonged time, it is preferred to use cells expressing the therapeutic protein in a stable manner. Stable expression requires the transformation of the polynucleotide encoding for the protein of selection together with a polynucleotide encoding for a protein which allows selecting against those cells which have not been incorporated into the marker. Suitable selection systems are, without limitation, thymidine kinase from herpes virus, hypoxanthine-guanine phosphoribosyltransferase, adenine phosphoribosyltransferase, genes encoding for proteins conferring resistance to an anti-metabolite such as dihydrofolate reductase, pyruvate transaminase, neomycin resistance gene and hygromycin resistance gene.

Suitable promoters for the expression of the therapeutic proteins include but are not necessarily limited to constitutive promoters such as those derived from eukaryotic virus genomes such as polyomavirus, adenovirus, SV40, CMV, avian sarcoma virus, hepatitis B virus, the metallothionein gene promoter, the herpes simplex virus thymidine kinase gene promoter, LTR regions of retroviruses, the immunoglobulin gene promoter, the actin gene promoter, the EF-1 alpha gene promoter, as well as inducible promoters in which the expression of the protein depends on the addition of a molecule or on an exogenous signal, such as the tetracycline system, the NFkappaB/UV light system, the Cre/Lox system and the heat shock gene promoter.

In principle, the exogenous genetic material can be inserted into the cells which are to be encapsulated by means of any method known in the art. The gene or the vector containing the gene can thus be administered by means of electroporation, transfection using polycationic proteins or liposomes or by using viral vectors, including adenoviral and retroviral vectors, and also non-viral vectors.

In a particular embodiment, the protein expressed by the cells is erythropoietin or a variant thereof. Particularly, suitable erythropoietins are human erythropoietin as well as variants thereof with improved activity and/or stability, such as the variants described in international patent application WO/2004/043382, or the variant known as novel erythropoiesis-stimulating protein (NESP or darbepoietin alpha).

The particles and microcapsules of the invention have the property of secreting the proteins produced by the cells located therein, such that if the protein is of therapeutic interest, they can be used for the treatment of those diseases requiring a supply of the therapeutic protein in question. Therefore, in another aspect the invention relates to pharmaceutical compositions containing the particles and microcapsules of the invention as well as to methods for the treatment of different diseases using the particles and microcapsules of the invention and to methods for preparing medicinal products which can be used for the treatment of diseases requiring the administration of the therapeutic proteins produced by the particles and microcapsules.

There is virtually no limit as to the type of disease that can be treated using the particles and microcapsules of the invention provided that there is a protein having a therapeutic effect on said disease. Thus, Table 1 includes a number of diseases which can be treated with the microcapsules of the invention, indicating the therapeutic protein necessary for such treatment.

**Table 1: Therapeutic proteins and diseases for which they can be used**

| Therapeutic protein | Disease |
|---|---|
| GHRH | Growth delays |
| GnRH | Prostate cancer, infertility |
| TRH | Cholestasis, hypothyroidism |
| Somatostatin | Treatment of bleeding of the digestive tract due to the rupture of esophageal varices |
| GH | GH deficiency, Turner syndrome, chronic renal failure, intrauterine growth retardation |
| LH | Infertility |
| FSH | Infertility |
| Prolactin | Prolactin deficiency |
| Oxytocin | Uterine stimulation in assisted birth |
| ADH | Diabetes insipidus, von Willebrand disease |
| Melatonin | Circadian rhythm sleep disorders, migraines |
| Müllerian inhibiting factor | Endometriosis, adenomyosis, uterine cancer |
| Calcitonin | Osteoporosis, hypercalcemia |
| Secretin | Autism |
| IGF-I | Growth delay, diabetes type I and II, amyotrophic lateral sclerosis |
| PNA | Hypertension |
| Insulin | Diabetes |
| Glucagon | Hypoglycemia |
| Parathyroid hormone | Osteoporosis |
| Coagulation factors | Hemophilia |
| IL-1 | Hematopoiesis stimulation |
| Interferons | Cancer |
| G-CSF | Cancer |
| BMPs | Neutropenia |
| TGF-beta | Spina bifida |
| Cyclosporine | Cancer |
| Hirudine | Autoimmune diseases, transplant rejections |
| Imiglucerase | Thrombosis |
| Tissue plasminogen activator (tPA) | Gaucher's disease |
| Alpha 1-antitrypsin | Congenital alpha 1-antitrypsin deficiency |
| Lactoferrin | Infections |
| Immunoglobulins | Infectious diseases |
| Superoxide dismutase | Amyotrophic lateral sclerosis |
| NGF, GDNF, BDNF, IGF | Neurodegenerative diseases |
| VEGF, FGF, angiopoietins | Angiogenesis |
| Endostatin, mAb VEGF | Anti-angiogenesis |
| mAb | Immunotherapy |

In a particular embodiment, the protein which is secreted by the protein is erythropoietin, in which case the microcapsules can be used for the treatment and prevention of diseases characterized by a low level of red blood cells or a deficient production of red blood cells. Examples of such diseases are anemia associated to chronic renal failure, anemia related to AZT therapy in AIDS patients, anemia in patients with non-myeloid type malignant diseases receiving chemotherapy, anemia associated to cancer, anemia associated with chronic inflammatory diseases (rheumatoid arthritis), anemia in patients who have undergone a surgical treatment to eliminate the need for allogeneic transfusions, sickle cell anemia, thalassemia, anemia associated to cystic fibrosis, menstrual disorders, acute losses of blood, anemia resulting from radiotherapy, the reduction of oxygen uptake associated to altitude, schizophrenia and neurodegenerative diseases.

After the microcapsules of the invention are implanted in a subject, they become vascularized, which assures their permanence in the tissue for a long time. Furthermore, due to the existence of interactions between the polymeric matrix and the cells, they are functional for more time. For both reasons, the microcapsules are capable of producing therapeutic protein in a stable manner for prolonged times, i.e., they act as sustained release medicinal products. Therefore, the microcapsules of the invention are especially useful for the expression of proteins with a short serum half-life, since the quick elimination of the proteins is compensated with stable and continued secretion thereof by the microcapsules. This allows preventing the continuous and repeated administration of a therapeutic agent in patients who require baseline levels of said agent for a prolonged time. For this reason, in a preferred embodiment the microcapsules are used for the expression of therapeutic proteins with a short elimination half-life.

The elimination half-life of a drug is the time necessary for the amount of said drug or xenobiotic agent present in the body (or in blood plasma) to be reduced to half by means of different elimination processes including degradation and renal excretion. The term "short" in relation to the elimination half-life indicates that the therapeutic protein has a half-life of less than 24, 20, 15, 10, 7, 5, 3 or 1 hour(s).

The microcapsules of the invention can be administered to the patient by parenteral and non-parenteral administration, including intravascular, intraperitoneal, subcutaneous, intradermal administration or by inhalation.

Due to the capacity of the microcapsules to release the therapeutic proteins produced by the encapsulated cells, the microcapsules are very useful as a device for the release of peptide compounds with biological activity. Therefore in another aspect, the invention relates to the use of the microcapsules of the invention for the release of peptide compounds with biological activity. In a preferred embodiment, said compound has a short serum elimination half-life. In another preferred embodiment, the release occurs in a controlled manner under the application of an external stimulus.

In another aspect, the invention relates to a method of preparing the particles of the invention comprising the steps of
a. Contacting a polymeric substance which is modified with a peptide Y with at least one cell having on its surface specific binding sites for said peptide Y, and
b. applying conditions allowing the formation of particles of polymeric material having less than 1 mm in diameter.

The necessary conditions in the second step for promoting aggregation of the polymers and giving rise to particles of less than 1 mm in diameter will depend on the type of polymer being used. In the case of thermoplastic polymers, the formation of the particles requires heat. In the event that the polymer is alginate, the formation of particles requires contacting the polymer-cell mixture with a divalent cation. The divalent cation is preferably calcium and the step of contacting the alginate-cell solution with the divalent cation solution is done by means of gas or liquid phase extrusion of the polymer-cell mixture on the divalent cation solution or by means of applying a voltage.

In another aspect, the invention provides a method for preparing the microcapsules of the invention comprising the steps of
a. Contacting a polymeric substance which is modified with a peptide Y with at least one cell having on its surface specific binding sites for said peptide Y, and
b. applying conditions allowing the formation of particles of polymeric material having less than 1 mm in diameter.
c. contacting the particles formed in step b with a material forming a membrane around the microcapsule,
wherein steps (a) and (b) essentially correspond with the steps of the method for preparing the particles of the invention.

In the third step, the polymer-cell particles are coated with a membrane conferring mechanical strength to the particles and acting as an immunological barrier. The coating process is carried out by means of contacting the microparticle with the coating material. In a preferred embodiment, in the event that the particles are alginate particles and the material forming the outer membrane is a polycationic material, the simple contact of the particles with poly-L-lysine allows the coating membrane to form due to the direct interaction occurring between the positive charge of the poly-L-lysine and the negative charge of the alginates.

In another preferred embodiment of the invention, in the event that the particles contain an additional outer layer, the method for preparing them includes an additional step in which the microcapsules are coated with said additional layer. Preferably, when the microcapsules are surrounded by a membrane of a polycationic material and the additional layer is alginate, the additional step simply requires contacting the microcapsules with the alginate solution, since the alginates, through their negative charge, are able to interact with the membrane made of polycationic material.

The invention is illustrated below based on the following examples provided by way of a non-limiting illustration of the scope thereof.

### EXAMPLES

### Example 1

The molecular weight of the alginate molecules rich in guluronic acid residues (Protanal LF240 D, FMC Technologies) was adjusted by means of irradiation with a cobalt 60 source. The irradiation doses ranged from 2 to 5 Mrads. The final molecular weight of the alginate molecules was analyzed by means of gel filtration chromatography (Viscotek).

After irradiation, the alginate molecules were modified with synthetic oligopeptides containing the (glycine)₄-arginine-glycine-aspartic acid-tyrosine sequences (GGGGRGDY, Commonwealth Biotechnology Inc.) using to that end aqueous carbodiimide reactions [1]. Briefly, alginate solutions were prepared in a 2-[N-morpholino]ethanesulfonic acid hydrate buffer (MES, Sigma) which were subsequently mixed with N-hydroxysulfosuccinimide (sulfo-NHS, Pierce Chemical), and 1-ethyl-3-[3-(dimethylamino)propyl]carbodiimide (EDC, Sigma) and the oligopeptides. After reacting for 24 hours, the modified alginate molecules were purified using filters (Fisher) to remove the molecules smaller than 3.500 g/mol. After the filtration, sterilization and lyophilization, the modified alginate was reconstituted to the desired concentration in the cell culture medium.

The viscosity of both binary gels, the solution with RGD alginate and the solution with unmodified alginate were checked and it was observed that both exhibit similar viscosity values (Figure 1a).

### Example 2

### Adhesive and biomimetic effects of RGD alginates

To evaluate the adhesive and biomimetic effects of RGD alginates, erythropoietin (EPO)-secreting mouse C2C12 skeletal myoblasts modified by genetic engineering were selected as candidate cells and mixed with the binary polymer solution. It was observed that both the RGD concentration and the alginate composition could control the phenotype of the myoblasts. These cells can be easily handled and once encapsulated and implanted in the host, they irreversibly come out of the cell cycle and fuse in multinucleated myofibrils. The latter is essential for controlling the therapeutic dose and for preventing possible biosafety risks. EPO was selected as model drug due to its resulting therapeutic effects and because it is easy to monitor its *in vivo* expression and bioactivity by following its hematocrit level. Furthermore, due to its weak pharmacokinetics, it is expected that cell encapsulation technology can prevent the continuous and repeated injection of EPO which is currently practiced.

Three-dimensional microcapsules were manufactured with RGD alginate and unmodified alginate solutions (Figure 1b). Uniform microcapsules with a small diameter (450 µm) were produced to assure optimal biocompatibility, long-term functionality and zero order release kinetics of EPO,.

To evaluate and compare the mechanical integrity of the RGD alginate and unmodified alginate microcapsules, the expansion behavior and the mechanical strength of the capsules were studied against compression. The expansion of the RGD alginate microcapsules (1.12 ± 0.01) was significantly lower than that of the systems of non-modified alginate (1.16 ± 0.01) (n=20, P<0.05) (Figure 1c), whereas the strength of the capsules under compression was significantly higher (49.6 ± 6.7 vs. 39.1 ± 4.7; n= 20, P < 0.01) (Figure 1d). These results support the idea of the interaction of myoblasts with the adhesion ligands which provides an additional mechanical integrity to the scaffolds with the cells acting as cross-linking agents.

### Example 3

### Viability and functionality of the myoblasts in the RGD alginate matrixes

To characterize the viability and functionality of the myoblasts in the matrixes, the cell metabolic activity and the release of EPO were studied *in vitro* during a 3-week period. These studies did not show major differences between the two types of microcapsules in cell viability or in the release of EPO in the short term. After 21 days in culture, the EPO secretion of RGD alginate microcapsules loaded with 100 cells measured was 71±9 IU of EPO/24 h, whereas the production in non-modified alginate matrixes was 72±5 IU EPO/24 h. These results suggest that the cells adapt satisfactorily to the new microenvironments. However, for the purpose of evaluating the possible impact of matrixes bioactivated with RGD on the survival and long-term functionality of encapsulated cells, 0.2 mL of both types of microcapsules (RGD alginate, 5 x 106 cells/mL alginate) were implanted in subcutaneous tissue of immunocompetent Balb/c mice (n=5) without implementing immunosuppression protocols (Figure 2c). The results indicate that the hematocrit of all the animals implanted (either with RGD microcapsules or without RGD) significantly increased (P<0.01) compared with the control mice, which showed a constant baseline level during the study. The mice in which the systems with RGD alginate were implanted increased their hematocrit level until day 28 (88.2%) and after that they maintained an asymptotic level close to 80% until the end of the study. The recipients with unmodified microcapsules showed a similar behavior until day 120. From that time on, a progressive drop in the hematocrit of the animals was detected. Moreover, in the last 100 days of the study the hematocrit level of the recipients with RGD alginate matrixes was significantly higher that that of the unmodified alginate systems (P<0.01) (Figure 2a). These results indicate that the matrixes bioactivated with RGD show a more sustained functionality, maintaining the hematocrit level close to 80% for 10 months after the administration of a single dose. In contrast, the particles of alginate not modified with RGD were only capable of maintaining the hematocrit level for 100 days (Orive, G. et al. 2005, Mol. Therapy, 12:283-289).

### Example 4

### Histological and functionality ex vivo study of the microcapsules

The microcapsules were explanted from all the recipients 330 days after the beginning of the study. At the time of the explantation, both the capsules with RGD and without RGD had formed a neovascularized and irregular structure which was totally adhered to the subcutaneous tissue. The microcapsules were separated from the aggregates in a simple manner and still maintained the spherical shape and the defined semi-permeable membrane (Figure 2d). To determine the functionality of the cells included in said microcapsules, the amount of EPO released from both systems was measured (Figure 2e). The results confirm that the systems with RGD released more EPO than the capsules without modified alginate (P<0.05).

The histological analysis of the capsules showed a minimum inflammatory reaction based on a thin layer of fibroblasts surrounding both types of capsules (Figure 2f) despite that commercial purified alginates were used for the experiments. It has been seen that the actual implantation process attracts and activates the cells of the immune system and stimulates the release of cytokines. However, the minimum immune reaction observed does not alter the functionality of the capsules. The fact that EPO inhibits some pro-inflammatory cytokines including IL-6, TNF-alpha and MCP-1, which exerts deleterious effects and triggers failure in the transplant, is interesting.

Another important histological observation was the presence of a high number of capillaries surrounding the aggregates of RGD microcapsules (Figure 2f). The formation of a capillary network around the capsules reduces the distance between the cells and the vasculature to the minimum, assuring a better nutritional and oxygen supply and thus improving the long-term functionality of the systems.

### Example 5

### 5.1 Effect of cell density on the activity of the microcapsules

A dose-response study was performed to evaluate the potential versatility of the RGD capsules for the controlled release of drugs. RGD matrixes were manufactured with different cell densities (5; 3.75; 2.5 and 1.25 x 10⁶ cells/mL alginate) and therefore, with different dosages of EPO (Figure 3a). The studies of *in vitro* metabolic activity and of EPO production clearly showed a dose-dependent relationship (Figure 3b, 3c). To evaluate the therapeutic potential of the invention, 0.2 mL of different RGD matrixes were subcutaneously implanted in Balb/c mice. The results indicate that the hematocrit of all the recipients implanted with the RGD microcapsules, independently of the dose, were significantly higher than in control mice for all the time periods (P<0.05). The hematocrit level of all the mice transplanted with the RGD microcapsules reached a peak on day 28 (Figure 3d) from a baseline value (46 ± 7). At this point, the hematocrit was 87 ± 3; 88 ± 4; 86.4 ± 1.5; 81 ± 8 % for the cell densities of 5; 3.75; 2.5 and 1.25 x 10⁶.

A dose-dependent effect was observed only in the first 14 days. From day 28 until day 60, the hematocrit value obtained with all the formulations was not significantly different, indicating that maximum hematopoietic effect is obtained with all the formulations. In the rest of the time periods, the mice received a higher dose showing hematocrit values significantly higher than those of the recipients which received a lower dose (P<0.05). From this day until the end of the study, the hematocrit of the mice with the lower dose-response decreased progressively whereas the other 3 formulations maintained their hematocrit. Despite the fact that a lower dose of EPO would be advisable for the treatment of anemia, higher doses would be used for the treatment of schizophrenia or for neuroprotection.

### 5.2 Ex vivo histological analyses of the microcapsules.

All the RGD microcapsules were explanted 200 days after implantation. The microcapsules again formed irregular aggregates surrounded by vascular networks (Figure 3e). Interestingly, the vascularization of the aggregates also seems to be dose-dependent, with less formation of capillaries or even without any formation in the case of the RGD capsules with lower cell density (1.25 x 10⁶).

The results shown herein indicate that a minimum dose of EPO would be necessary to stimulate the capillary formation around the capsule aggregates.

The histological analysis of the RGD matrixes showed a lower inflammatory response and a capillary network around the capsule aggregates (Figure 3f). The microcapsules present in the aggregates remained intact with a defined and uniform semi-permeable membrane. After isolating the matrixes from the aggregates, it was observed that all the encapsulated cells remained viable and released detectable amounts of EPO.

## Claims

1. A particle with a maximum diameter of 1 mm comprising:
a) a polymer X functionalized with at least one peptide Y;
b) at least one cell having on its surface specific binding sites for said peptide Y, wherein the cell is bound to polymer X by means of the interaction between peptide Y and the specific binding sites for said peptide Y on the surface of the cell.

2. The particle according to claim 1, wherein said polymer X is alginate.

3. The particle according to claims 1 or 2, wherein peptide Y is a peptide containing the RGD sequence.

4. A microcapsule comprising
a) a particle according to any of the previous claims and
b) a semi-permeable membrane surrounding the particle

5. The microcapsule according to claim 4, wherein said membrane is a polylysine membrane.

6. The microcapsule according to claims 4 or 5, containing, surrounding the membrane on the outside thereof, a second membrane made of a material inhibiting cell adhesion.

7. The microcapsule according to claim 6, wherein the second membrane is made of alginate.

8. The particle according to any of claims 1 to 3, or the microcapsule according to any of claims 4 to 7, wherein the cells are genetically modified cells.

9. The particle or microcapsule according to claim 8, wherein said cells express a peptide compound with biological activity.

10. The particle or microcapsule according to claim 9, wherein said cells express a peptide compound with biological activity under the control of a constitutive or inducible expression promoter.

11. The particle or microcapsule according to any of claims 8 to 10, wherein said compound is erythropoietin (EPO).

12. The particle or microcapsules according to any of claims 8 to 11 for the use thereof in medicine.

13. Use of a particle or microcapsule according to any of claims 8 to 11 for preparing a medicinal product for the treatment and prevention of diseases in which a supply of the peptide compound with biological activity which is expressed by the cells forming part of the microcapsules is required.

14. The use according to claim 13, wherein the peptide compound has a short elimination half-life.

15. The use according to claim 14, wherein the peptide compound with a short serum half-life is erythropoietin and the disease to be treated or prevented is a disease selected from the group of anemia associated to chronic renal failure, anemia relating to therapy with AZT in AIDS patients, anemia in patients with non-myeloid type malignant diseases receiving chemotherapy, anemia associated to cancer, anemia associated with chronic inflammatory diseases, particularly rheumatoid arthritis, who have undergone a surgical treatment to eliminate the need for allogeneic transfusions, sickle cell anemia, thalassemia, anemia associated to cystic fibrosis, menstrual disorders, acute losses of blood, anemia resulting from radiotherapy or from a reduced oxygen uptake associated to altitude, schizophrenia and neurodegenerative diseases.

16. Use of a particle or microcapsule according to any of claims 8 to 11 as a device for the release of a peptide compound with biological activity.

17. The use according to claim 16, wherein said compound is a compound showing a short elimination half-life.

18. The use according to claim 16 or 17, wherein said release occurs in a controlled manner under the application of an external stimulus.

19. A method for producing a particle according to any of claims 1 to 3, comprising the steps of
a. Contacting a polymeric substance which is modified with a peptide Y with at least one cell having on its surface specific binding sites for said peptide Y, and
b. applying conditions allowing the formation of particles of polymeric material having less than 1 mm in diameter.

20. The method according to claim 19, wherein the polymeric substance modified with peptide Y is an alginate and the conditions used in step (a) consist of contacting the modified polymer and cell mixture with a divalent cation solution.

21. A method for preparing a microcapsule according to any of claims 4 to 7 comprising the steps of
a. Contacting a polymeric substance which is modified with a peptide Y with cells having the capacity to bind to said peptide Y
b. applying conditions allowing the formation of particles of polymeric material having less than 1 mm in diameter
c. coating the microparticle formed in step (b) with a membrane made of a material different from the polymer used in step (a).

22. The method according to claim 21, wherein the polymeric substance modified with peptide Y is an alginate and the conditions used in step (a) consist of contacting the modified polymer and cell mixture with a divalent cation solution.

23. The method according to any of claims 21 or 22, additionally comprising the step of coating the microcapsules with an additional layer of material different from the material forming the membrane formed during step (c).

24. The method according to claim 23, wherein the additional layer contains mostly alginates.
